# EUROPEAN PATENT SPECIFICATION

(11) **EP 0 503 988 B1**
(45) Date of publication and mention of the grant of the patent: **15.03.2000**
(21) Application number: 92400384.1
(22) Date of filing: 13.02.1992
(51) Int. Cl.: A61K 31/135, A61K 47/10

(54) **Compositions to treat onychomycoses**
Arzneizubereitungen zur Behandlung von Onychomykosen
Compositions pour le traitement des onychomycoses

(30) Priority: 08.03.1991 FR 9102838
(43) Date of publication of application: 16.09.1992
(73) Proprietor: Novartis AG, 4058 Basel (CH); Novartis-Erfindungen Verwaltungsgesellschaft m.b.H., 1230 Wien (AT)
(72) Inventor: Laugier, Jean-Pierre, F-92160 Antony (FR); Rude, Marie-France, F-94800 Villejuif (FR); Touzan, Philippe, F-31520 Ramonville Saint Agne (FR); Ringenbach, François, F-92220 Bagneux (FR)

(56) References cited:
- EP-A- 0 298 271
- EP-A- 0 399 858
- WO-A-87/02580
- FR-A- 2 572 933
- GB-A- 2 197 194

## Description

La présente invention porte sur l'utilisation d'agents de pénétration hydrophiles dans des compositions dermatologiques pour le traitement, chez l'homme et l'aminal, des onychomycoses notamment à dermatohpytes ou à Candida, ainsi que sur des compositions dermatologiques renfermant de tels agents de pénétration.

Zaias et coll., Clinical Experimental Dermatology 1989, 14, 120-123 ont décrit le traitement des onychomycoses à Trichophyton rubrum par l'administration de terbinafine par voie orale. Cet agent antifongique, original par sa structure et par son activité sur les champignons dermatophytes, présente une faible toxicité par voie orale et permet de mettre en oeuvre avec succès des traitements à long terme des onychomycoses. Il est apparu ainsi que le traitement le plus efficace consiste en l'administration de 250 mg/jour de terbinafine par voie orale pendant 6 mois. Ceci représente un progrès par rapport aux médicaments connus antérieurement, à savoir le kétoconazole et la griséofulvine. Toutefois, l'administration de la terbinafine présente des inconvénients, à savoir que, d'une part, la durée du traitement reste très longue, avec un risque élevé de non-observance, et, d'autre part, le coût du traitement est extrêmement élevé, car il est lié à sa durée et à la prise quotidienne de 250 mg de terbinafine.

C'est pourquoi on cherche depuis longtemps à traiter les onychomycoses par voie transunguéale. Le problème, qui se pose alors, est celui d'assurer la pénétration des agents antifongiques dans l'ongle et sous l'ongle; de tels agents antifongiques pourraient alors avantageusement être ceux de la famille des allylamines à laquelle appartient la terbinafine, celle-ci étant à ce jour le seul composé de la famille utilisable par voie orale.

A cet égard, il est proposé, par le brevet européen EP-A-0 064 830, l'administration, par voie transunguéale, de solutions concentrées dans des acides gras en C₈-C₂₀ dont l'acide undécylénique, d'agents antifongiques liposolubles appartenant à la famille des imidazoles, ces solutions pouvant encore être solubilisées dans un milieu solvant choisi parmi les alcanols inférieurs, tels que l'éthanol et l'isopropanol, les esters comme l'acétate d'éthyle, et les cétones, comme l'acétone et la butanone-2. Les acides gras en C₈-C₂₀ mentionnés jouent le rôle d'agents de pénétration transunguéale,l'acide undécylénique servant aussi à renforcer les propriétés antifongiques des imidazoles. Une composition de ce type est commercialisée sous la dénomination "TROSYD solution", à 28% de tioconazole, pour compléter les traitements oraux par la griséofulvine.

On connaît également, par le brevet français FR-B-2 523 447, des préparations thérapeutiques antimycotiques à base de crème ou d'onguent, contenant une ou plusieurs matières actives antimycotiques, ainsi que de l'urée à raison de 5-20% en poids par rapport au poids total de la préparation.

L'état de la technique montre aussi que,tout en cherchant a augmenter la pénétration d'agents antifongiques, par eux-mêmes peu diffusibles dans l'ongle, on propose des compositions de type "vernis à ongles" comportant à cet effet un composant polymérique et devant permettre une libération programmée de l'agent actif.

Ainsi, le brevet japonais JP-01-110 620 décrit une composition antifongique destinée à être utilisée pour les ongles, et comprenant du poly(acétate de vinyle), un agent antifongique, de préférence un imidazole, des esters d'acides gras à longue chaîne en C₈-C₂₀ et/ou des alcools supérieurs, ainsi qu'un solvant volatil.

On connaît aussi, par le brevet européen EP-A-319 964, des préparations antifongiques du type filmogène pour l'application externe, qui comprennent environ 0,1-1,5% de tolnaphtate, environ 10-20% d'un copolymère méthacrylate de diméthylaminoéthyle-ester d'acide méthacrylique, et environ 0,5-10% d'un ester d'acide gras à chaîne moyenne, dans un solvant alcoolique, mais ne contenant pratiquement pas d'eau. Le tolnaphtate est relargué progressivement à partir de la matrice polymérique et la pénétration est favorisée par l'ester d'acide gras, en milieu anhydre.

Par le brevet allemand DE-A-3 544 983, on connaît également des vernis antimycotiques pour les ongles, renfermant un agent filmogène insoluble dans l'eau, un agent antifongique de la série de l'hydroxy-1 pyridone-2, tel que le cyclopirox ou l'octopirox, et un solvant exclusivement anhydre.

Par la demande internationale WO 87/02580, on connaît un véhicule pharmaceutique capable de délivrer à l'ongle un principe actif, formé d'un polymère hydrophile et d'un solvant pharmaceutiquement acceptable de ce polymère hydrophile. L'exemple 2 mentionne l'association d'un agent antifongique avec un polymère hydrophile dans un véhicule à base d'éthanol et d'eau. L'agent antifongique doit avoir, de préférence, un poids moléculaire inférieur à 300 et une solubilité dans l'eau au moins égale à 0,01% en poids.

On connaît également, par la demande de brevet allemand DE-A-3 720 147, des vernis pour le traitement des onychomycoses, contenant un polymère filmogène insoluble dans l'eau, une substance antifongique de type imidazole, tolnaphtate, chlorhydrate de naftifine, et un solvant du polymère filmogène choisi parmi les solvants organiques habituellement utilisés dans le domaine cosmétique pour réaliser les vernis à ongles, avec un point d'ébullition bas, inférieur à 100°C. L'exemple 8 de cette demande de brevet concerne un vernis renfermant un agent antifongique de la famille des allylamines (chlorhydrate de naftifine), de l'eau à raison de 4%, de poly(butyrate de vinyle) comme polymère filmogène, de la nitrocellulose et de l'acétate d'éthyle.

Ainsi, l'état de la technique présente des compositions à base d'un agent antifongique de bas poids moléculaire, dans un mélange hydro-alcoolique permettant de solubiliser ledit agent antifongique, avec ajout d'un polymère hydrophile, comme dans le cas de la demande internationale WO 87/02580, ou d'un polymère insoluble dans l'eau, comme c'est- le cas dans la demande de brevet DE-A-3 720 147. Toutefois, aucune de ces compositions ne donne réellement de bons résultats. Ainsi les formulations, telles que celles décrites dans l'exemple 8 de la demande de brevet allemand DE-A-3 720 147 présentent de nombreux inconvénients qui ne permettent pas, dans le traitement des onychomycoses, d'atteindre les buts souhaités :
- en premier lieu, les polymères utilisés empêchent le relargage du principe actif : après évaporation des solvants, le réseau polymérique retient la naftifine et empêche sa pénétration dans la couche cornée ;
- lors d'une deuxième application, il se produit une accumulation du couple polymère + principe actif à la surface de la première couche, d'où la non-pénétration du principe actif ;
- la nitrocellulose est un puissant oxydant et la naftifine est instable chimiquement dans ce milieu et donc inactivée ; et
- l'acétate d'éthyle est bien connu pour déshydrater l'ongle et donc ralentir la pénétration de l'antifongique ; c'est en effet un solvant lipophile qui va à l'encontre de l'effet désiré.

Cherchant une solution au problème posé, la Société déposante a constaté que les agents de pénétration hydrophiles utilisés pour favoriser la pénétration transcutanée d'agents actifs ont la propriété d'augmenter de façon considérable la teneur en agent antifongique dans l'ongle et sous l'ongle.

Ce résultat est tout à fait inattendu, car de nombreuses études scientifiques ont mis en évidence que l'ongle est de nature hydrophile, :c'est-à-dire qu'il se comporte comme un hydrogel contrairement au stratum corneum de la peau, qui est de nature lipophile et qui se comporte comme une barrière à l'eau. De ce fait, il est surprenant que les agents de pénétration couramment utilisés pour traverser le stratum corneum favorisent la pénétration d'agents antifongiques dans l'ongle, comme cela est mis en évidence dans l'exemple 10 ci-après.

La présente invention porte sur une composition dermatologique destinée au traitement des onychomycoses, se présentant sous forme d'une solution et comprenant, une quantité efficace de chlorhydrate de terbinafine comme agent antifongique, une quantité efficace d'au moins un agent favorisant la pénétration par ledit agent antifongique de la tablette unguéale kératinisée de l'ongle, et un milieu solvant dudit agent antifongique, caractérisée par le fait que ledit agent antifongique est au moins partiellement soluble dans l'eau, que ledit milieu solvant comporte de l'eau en mélange avec au moins un tiers-solvant choisi parmi les alcanols en C₂ - C₈ à chaîne droite ou ramifiée, et que ledit (ou lesdits) agent(s) de pénétration est (ou sont) hydrophile(s) et au moins partiellement miscible(s) audit milieu solvant.

Le chlorhydrate de terbinafine a pour formule : 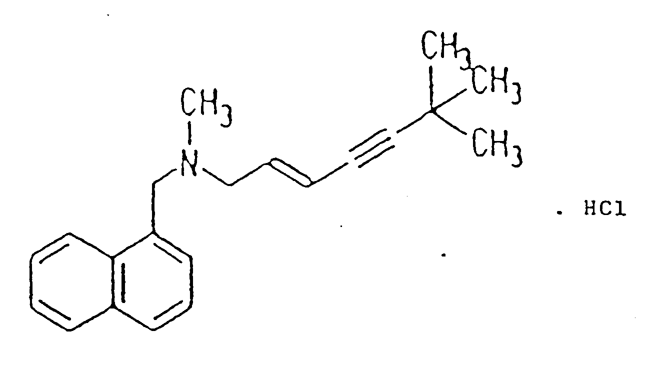 (chlorhydrate de terbinafine)

La concentration en agent antifongique est notamment d'environ 2 à 30% en poids et de préférence 5 à 20% en poids par rapport au poids total de la composition.

Les agents de pénétration hydrophiles sont avantageusement choisis parmi :
- les glycols, comme, par exemple, le propylène glycol, le butylène glycol, l'hexylène glycol, l'éthylène glycol et les polyéthylènes glycols ;
- les monoéthers de glycols comme, par exemple, les monoéthers de l'éthylène glycol commercialisés sous les dénominations "DOWANOL EE, EM, EB, DE, DM, DB", et les monoéthers du propylène glycol commercialisés sous les dénominations "DOWANOL PM, DPM, TPM, PnB, PPH, DPnB, TPnB, PMA";
- les diéthers de glycols, comme par exemple ceux commercialisés sous la dénomination de "PROGLYDES®", en particulier l'éther diméthylique du propylène glycol et notamment l'éther diméthylique du dipropylène glycol,
- le diméthylsulfoxyde, le caprolactame, le diméthylisosorbide, l'isopropylidène glycérol, la diméthylimidazolidinone, le N-methyl-pyrrolidone-2, la pyrrolidone-2, le lactate d'éthyle, les glycérides en C₈-C₁₀ polyoxyéthylénés, notamment ceux commercialisés sous la dénomination "LABRASOL®", le polyéthylène glycol 20 glycéryl laurate et le diméthylacétamide.

La concentration en agent(s) de pénétration va avantageusement d'environ 1 à 60 % en poids, par rapport au poids total de la composition.

Le milieu solvant est avantageusement constitué par de l'eau en mélange avec au moins un tiers solvant pris dans le groupe formé par les alcanols en C₂-C₈, à chaîne droite ou ramifiés. Comme tiers solvant, on peut citer, de préférence, l'éthanol, l'isopropanol et le n-butanol.

L'eau est présente notamment à raison d'environ 5 à 30 % en poids par rapport au poids total; quant à la concentration en tiers-solvant(s), elle va notamment d'environ 10 à 90 % en poids, par rapport au poids total de ladite composition.

De préférence, la composition selon l'invention est exempte de tensio-actif ; mais elle peut renfermer au moins un additif additif pris dans le groupe formé par les agents conservateurs, tels que l'alcool phényléthylique, l'alcool benzylique et le phénoxyéthanol; les antioxydants, tels que le butylhydroxyanisole (BHA), le butylhydroxytoluène (BHT), l'ascorbate de palmityle, l'a-tocophérol et/ou ses esters ; les colorants, charges ou pigments, tels que les micatitanes couramment utilisés dans le domaine cosmétique pour réaliser des vernis à ongles ; et les polymères capables d'empêcher l'écoulement de la composition avant pénétration, comme, par exemple, les dérivés alkylcellulosiques totalement solubles dans le milieu solvant utilisé et choisis, en particulier, parmi les méthylcelluloses, les éthylcelluloses et les hydroxyalkylcelluloses, telles que celles commercialisées sous la dénomination "KLUCEL".

La composition selon l'invention se présente avantageusement sous la forme d'une lotion ou d'un gel fluide.

La présente invention porte également sur un procédé de préparation d'une composition dermatologique selon l'invention.

Pour mieux faire comprendre l'objet de l'invention, on va en décrire maintenant, à titre d'exemples purement illustratifs et non limitatifs, plusieurs modes de mise en oeuvre :

### EXEMPLE 1 :

On réalise la formulation suivante :

| | |
|---|---|
| Terbinafine-HCl | 10% poids |
| Ethanol | 30% |
| Eau purifiée | 30% |
| Ether monoéthylique du diéthylène glycol | 30% |

On solubilise la terbinafine-HCl dans le mélange eau-éthanol, à 30°C, sous agitation, puis on ajoute progressivement l'éther monoéthylique du diéthylène glycol jusqu'à solubilisation complète. on applique la lotion ainsi obtenue sur les ongles malades, en vue du traitement d'une onychomycose, soit à l'aide d'un pinceau, soit à l'aide d'un pulvérisateur à pompe.

L'amélioration de l'état de l'ongle est visible dès le deuxième mois de traitement et se traduit par la repousse d'ongle sain, qui élimine progressivement l'ongle malade.

Dans chacun des exemples 2 à 8 suivants, on procède comme à l'exemple 1 et l'on obtient une lotion que l'on applique comme à l'exemple 1 ; dans l'exemple 9, on obtient un gel mais le mode d'application reste le même. On constate une amélioration significative de l'onychomycose dès le deuxième mois de traitement. Toutes les proportions sont données en poids par rapport au poids total de la composition.

### EXEMPLE 2 :

On prépare la composition suivante :

| | |
|---|---|
| Terbinafine-HCl | 15% |
| Isopropanol | 40% |
| Eau purifiée | 20% |
| Ether monométhylique du propylène glycol | 25% |

### EXEMPLE 3 :

On prépare la composition suivante :

| | |
|---|---|
| Terbinafine-HCl | 5% |
| n-Butanol | 75% |
| Ether monométhylique du tripropylène glycol | 5% |
| Eau purifiée | 15% |

### EXEMPLE 4 :

On prépare la composition suivante :

| | |
|---|---|
| Naftifine-HCl | 10% |
| Ethanol | 64% |
| Eau purifiée | 14% |
| Diméthylisosorbide | 12% |

### EXEMPLE 5 :

On prépare la composition suivante :

| | |
|---|---|
| Naftifine-HCl | 10% |
| Isopropanol | 30% |
| Eau purifiée | 30% |
| Isopropylidène glycérol commercialisé sous la dénomination "SOLKETAL" | 30% |

### EXEMPLE 6 :

On prépare la composition suivante :

| | |
|---|---|
| Naftifine-HCl | 5% |
| Isopropanol | 45% |
| Eau purifiée | 30% |
| Caprolactame | 30% |

### EXEMPLE 7 :

On prépare la composition suivante :

| | |
|---|---|
| Terbinafine-HCl | 5% |
| Eau purifiée | 15% |
| Ethanol | 56,8% |
| Diméthylsulfoxyde | 15% |
| Polyéthylène glycol 20 glycéryl laurate | 8% |
| Butylhydroxytoluène | 0,1% |
| Butylhydroxyanisole | 0,1% |

### EXEMPLE 8 :

On prépare la composition suivante :

| | |
|---|---|
| Terbinafine-HCl | 20% |
| Eau purifiée | 5% |
| Ethanol | 40% |
| Ether mono-n-butylique du tripropylène glycol | 5% |
| Diméthylsulfoxyde | 25% |
| Diméthyl imidazolidinone | 5% |

### EXEMPLE 9 :

On prépare la composition suivante :

| | |
|---|---|
| Terbinafine-HCl | 8 % |
| Eau purifiée | 25 % |
| Ether monométhylique du propylène glycol | 30 % |
| Ethanol | 63,25 % |
| Butylhydroxytoluène | 0,05 % |
| Hydroxypropyl cellulose | 0,2 % |

### EXEMPLE 10 : Mise en évidence du phénomène de pénétration dans l'ongle d'un agent antifongique, grâce à un agent de pénétration hydrophile.

On a mis en évidence le phénomène de pénétration susindiqué grâce à un modèle in vitro qui reproduit de façon remarquable l'ongle humain.

A cet effet, on place, à la surface de cellules de Franz (type statique), de fines rondelles de corne de sabot de boeuf, d'épaisseur constante de 200 µm à 400 µm. La couche cornée reçoit la composition antifongique, et le liquide de "survie", à la face inférieure, est utilisé pour effectuer le dosage de l'agent antifongique après un temps T donné.

Après application de la composition antifongique à étudier à la surface de la rondelle de corne, il est ainsi possible de doser, au bout du temps T, l'agent antifongique resté en surface, l'agent antifongique ayant pénétré dans la corne, et l'agent antifongique se trouvant dans le liquide de "survie", et de réaliser ainsi des bilans de pénétration.

Selon l'ivention, on a préparé une lotion, dont la composition (en % en poids) est indiquée ci-après :

| | | |
|---|---|---|
| • | Terbinafine-HCl | 10 |
| • | Eau purifiée | 30 |
| • | Ethanol | 30 |
| • | Ether monoéthylique du dipropylène glycol | 30 |

On a appliqué cette lotion, à raison de 5µl/cm² de corne, 1cm² de corne correspondant à 100mg de corne. Au bout de 72 heures de traitement, 0,122 ± 0,02% (pour n = 11 essais) de la quantité de terbinafine-HC1 appliquée est stocké dans la corne, soit environ 610ng, à savoir environ 6,10ng de terbinafine-HC1/mg de corne.

D'après Finlay et Coll, British Journal of Dermatology, 1990, 123, 481 à 486, les taux de terbinafine-HC1 dans l'ongle après un traitement oral pendant 8 semaines à raison de 250mg/jour -traitement réputé efficace-, ne sont que de 0,4 ± 0,15ng de terbinafine-HC1 par mg d'ongle. Il en résulte qu'un traitement topique unique pendant 72 heures de la lotion selon l'invention à 10% de terbinafine-HC1 délivre environ 10 à 25 fois plus d'agent actif qu'un traitement oral pendant 8 semaines à raison de 250mg/jour.

## Claims (Claims for the following Contracting State(s): AT, BE, CH, DE, DK, FR, GB, IT, LI, NL, PT, SE)

1. Dermatological composition intended for the treatment of onychomycoses, presented in the form of a solution and containing
- an effective quantity of terbinafine hydrochloride as antifungal agent,
- an effective quantity of at least one agent that favours the penetration of said antifungal agent through the keratinised ungual layer of the nail, and
- a solvent medium for the said antifungal agent,
characterised in that the said solvent medium contains water mixed with at least one co-solvent chosen from among the straight-chained or branched C₂-C₈-alkanols, and that the said penetration agent(s) is (or are) chosen from the group comprising the glycols, glycol monoethers, glycol diethers, dimethyl sulphoxide, caprolactam, dimethylisosorbide, isopropylidene glycerol, dimethylimidazolidinone, N-methylpyrrolidone-2, pyrrolidone-2, ethyl lactate, the polyoxyethylenated C₈-C₁₀-glycerides, polyethylene glycol 20 glyceryl laurate and dimethylacetamide.

2. Composition according to claim 1, characterised in that the concentration of antifungal agent is 2 to 30% by weight relative to the total weight of said composition.

3. Composition according to claim 1 or 2, characterised in that the glycols are taken from the group comprising propylene glycol, butylene glycol, hexylene glycol, ethylene glycol and the polyethylene glycols; the glycol monoethers are taken from the group comprising the monoethers of ethylene glycol and the monoethers of propylene glycol; and the glycol diethers are taken from the group comprising propylene glycol dimethyl ether and dipropylene glycol dimethyl ether.

4. Composition according to one of claims 1 to 3, characterised in that the penetration agent is dipropylene glycol dimethyl ether.

5. Composition according to one of claims 1 to 4, characterised in that the concentration of penetration agent is 1 to 60% by weight relative to the total weight of the composition.

6. Composition according to one of claims 1 to 5, characterised in that the water and the co-solvent(s) are present in proportions, respectively, of 5 to 30% by weight and 10 to 90% by weight, relative to the total weight of the composition.

7. Composition according to one of claims 1 to 6, characterised in that it contains at least one additive taken from the group comprising preservatives, antioxidants, colorants, charges, pigments and polymers intended to prevent running of the composition before penetration.

8. Composition according to one of claims 1 to 7, characterised in that it is present in the form of a lotion or a fluid gel.

## Claims (Claims for the following Contracting State(s): ES, GR)

1. Process for the preparation of a dermatological composition intended for the treatment of onychomycoses, characterised in that an effective quantity of terbinafine hydrochloride as antifungal agent is dissolved whilst stirring in a mixture consisting of water and a co-solvent chosen from among the straight-chained or branched C₂-C₈-alkanols, an effective quantity of penetration agent(s), chosen from the group comprising the glycols, the glycol monoethers, the glycol diethers, dimethyl sulphoxide, caprolactam, dimethyl isosorbide, isopropylidene glycerol, dimethyl imidazolidinone, N-methyl-pyrrolidone-2, pyrrolidone-2, ethyl lactate, the polyoxyethylenated C₈-C₁₀ glycerides, polyethylene glycol glyceryl laurate and dimethylacetamide, is gradually added, and a solution is obtained.

2. Process according to claim 1, characterised in that the quantity of antifungal agent(s) is such that the concentration thereof in the solution is from 2 to 30%, relative to the total weight of the solution.

3. Process according to claim 1 or 2, characterised in that the the glycols are taken from the group comprising propylene glycol, butylene glycol, hexylene glycol, ethylene glycol and the polyethylene glycols; the glycol monoethers are taken from the group comprising ethylene glycol monoethers and propylene glycol monoethers; and the glycol diethers are taken from the group comprising propylene glycol dimethyl ether and dipropylene glycol dimethyl ether.

4. Process according to any one of claims 1 to 3, characterised in that the penetration agent is dipropylene glycol dimethyl ether.

5. Process according to one of claims 1 to 4, characterised in that the solution obtained contains 1 to 60% by weight of penetration agent(s) relative to the total weight of the composition.

6. Process according to one of claims 1 to 5, characterised in that the solution obtained contains 5 to 30% by weight of water and 10 to 90% by weight of alkanol, relative to the total weight of the composition.

7. Process according to one of claims 1 to 6, characterised in that, at any stage of the process, at least one additive is added, and this is taken from the group comprising preservatives, antioxidants, colorants, charges, pigments and polymers intended to prevent running of the composition before penetration.

8. Dermatological composition intended for the treatment of onychomycoses, presented in the form of a solution and containing
- an effective quantity of terbinafine hydrochloride as antifungal agent,
- an effective quantity of at least one agent that favours the penetration of said antifungal agent through the keratinised ungual layer of the nail, and
- a solvent medium for the said antifungal agent,
characterised in that the said solvent medium contains water mixed with at least one co-solvent chosen from among the straight-chained or branched C₂-C₈-alkanols, and that the said penetration agent(s) is (or are) chosen from the group comprising the glycols, glycol monoethers, glycol diethers, dimethyl sulphoxide, caprolactam, dimethylisosorbide, isopropylidene glycerol, dimethylimidazolidinone, N-methylpyrrolidone-2, pyrrolidone-2, ethyl lactate, the polyoxyethylenated C₈-C₁₀-glycerides, polyethylene glycol 20 glyceryl laurate and dimethylacetamide.

## Revendications (Revendications pour l'(les) Etat(s) contractant(s) suivant(s): AT, BE, CH, DE, DK, FR, GB, IT, LI, NL, PT, SE)

1. Composition dermatologique destinée au traitement des onychomycoses, se présentant sous forme d'une solution et comprenant,
- une quantité efficace de chlorhydrate de terbinafine comme agent antifongique,
- une quantité efficace d'au moins un agent favorisant la pénétration par ledit agent antifongique de la tablette unguéale kératinisée de l'ongle et,
- un milieu solvant dudit agent antifongique,
caractérisé par le fait que ledit milieu solvant comporte de l'eau en mélange avec au moins un tiers-solvant choisi parmi les alcanols en C₂-C₈ à chaîne droite ou ramifiée, et que ledit (ou lesdits) agent(s) de pénétration est (ou sont) pris dans le groupe formé par les glycols, les monoéthers de glycols, les diéthers de glycols, le diméthylsulfoxyde, le caprolactame, le diméthylsisosorbide, l'isopropylidène glycérol, la diméthylimidazolidinone, la N-méthyl-pyrrolidone-2, la pyrrolidone-2, le lactate d'éthyle, les glycérides en C₈-C₁₀ polyoxyéthylénés, le poly-éthylène glycol 20 glycéryle laurate et le diméthylacétamide.

2. Composition selon la revendication 1, caractérisée par le fait que la concentration en agent antifongique est de 2 à 30% en poids par rapport au poids total de ladite composition.

3. Composition selon la revendication 1 ou 2, caractérisée par le fait que les glycols sont pris dans le groupe formé par le propylène glycol, le butylène glycol, l'hexylène glycol, l'éthylène glycol et les polyéthylène glycols; les monoéthers de glycols sont pris dans le groupe formé par les monoéthers de l'éthylène glycol et les monoéthers du propylène glycol; et les diéthers de glycols sont pris dans le groupe formé par l'éther diméthylique du propylène glycol et l'éther diméthylique du dipropylène glycol.

4. Composition selon l'une des revéndications 1 à 3, caractérisée par le fait que l'agent de pénétration est l'éther diméthylique du dipropylène glycol.

5. Composition selon l'une des revendications 1 à 4, caractérisée par le fait que la concentration en agent(s) de pénétration est de 1 à 60% en poids, par rapport au poids total de la composition.

6. Composition selon l'une des revendications 1 à 5, caractérisée par le fait que l'eau et le (ou les) tiers-solvant(s) sont présents à raison respectivement de 5 à 30% en poids et 10 à 90% en poids, par rapport au poids total de la composition.

7. Composition selon l'une des revendications 1 à 6, caractérisée par le fait qu'elle comprend au moins un additif pris dans le groupe formé par les agents conservateurs, les antioxydants, les colorants, les charges, les pigments et les polymères destinés à empêcher l'écoulement de la composition avant pénétration.

8. Composition selon l'une des revendications 1 à 7, caractérisée par le fait qu'elle se présente sous la forme d'une lotion ou d'un gel fluide.

## Revendications (Revendications pour l'(les) Etat(s) contractant(s) suivant(s): ES, GR)

1. Procédé de préparation d'une composition dermatologique destinée au traitement des onychomycoses, caractérisé par le fait que l'on solubilise sous agitation une quantité efficace de chlorhydrate de terbinafine comme agent antifongique dans un mélange formé d'eau et d'un tiers solvant choisi parmi les alcanols en C₂-C₈ à chaîne droite ou ramifiée, que l'on ajoute progressivement en quantité efficace un (des) agent(s) de pénétration pris dans le groupe formé par les glycols, les monoéthers deglycols,les diéthers de glycols, le diméthylsulfoxyde,lecaprolactame, le diméthylisosorbide, l'isopropylidène glycérol, la diméthylimidazolidinone, la N-méthyl-pyrrolidone-2, la pyrrolidone-2, le lactate d'éthyle, les glycérides C₈-C₁₀ polyoxyéthylénés, le polyéthylène glycol glycéryl laurate et le diméthylacétamide, et que l'on obtient une solution.

2. Procédé selon la revendication 1, caractérisé par le fait que la quantité d'agent(s)antifongique(s) est telle que dans la solution sa concentration soit de 2 à 30 % par rapport au poids total de la solution.

3. Procédé selon la revendication 1 ou 2, caractérisé par le fait que les glycols sont pris dans le groupe formé par le propylène glycol, le butylène glycol, l'hexylène glycol, l'éthylène glycol et les Polyéthylène glycols; les monoéthers de glycols sont pris dans le groupe formé par les monoéthers de l'éthylène glycol et les monoéthers du propylène glycol; et les diéthers de glycols sont pris dans le groupe formé par l'éther diméthylique du propylène glycol et l'éther diméthylique du dipropylène glycol.

4. Procédé selon l'une quelconques des revendications 1 à 3,caractérisé par le fait que l'agent de pénétration est l'éther diméthylique du dipropylène glycol.

5. Procédé selon l'une des revendications 1 à 4, caractérisé par le fait que la solution obtenue contient 1 à 60 % en poids d'agent(s) de pénétration par rapport au poids total de la composition.

6. Procédé selon l'une des revendications 1 à 5, caractérisé par le fait que la solution obtenue contient 5 à 30 % en poids d'eau et 10 à 90 % en poids d'alcanol, par rapport au poids total de la composition.

7. Procédé selon l'une des revendications 1 à 6, caractérisé par le fait que l'on ajoute à un stade quelconque du procédé au moins un additif pris dans le groupe formé par les agents conservateurs, les antioxydants, les colorants, les charges, les pigments et les polymères destinés à empêcher l'écoulement de la composition avant pénétration.

8. Composition dermatologique destinée au traitement des onychomycoses, se présentant sous forme d'une solution et comprenant,
- une quantité efficace de chlorhydrate de terbinafine comme agent antifongique,
- une quantité efficace d'au moins un agent favorisant la pénétration par ledit agent antifongique de la tablette unguéale kératinisée de l'ongle et,
- un milieu solvant dudit agent antifongique,
caractérisé par le fait que ledit milieu solvant comporte de l'eau en mélange avec au moins un tiers-solvant choisi parmi les alcanols en C₂-C₈ à chaîne droite ou ramifiée, et que ledit (ou lesdits) agent(s) de pénétration est (ou sont) pris dans le groupe formé par les glycols, les monoéthers de glycols, les diéthers de glycols, le diméthylsulfoxyde, le caprolactame, le diméthylsisosorbide, l'isopropylidène glycérol, la diméthylimidazolidinone, la N-méthyl-pyrrolidone-2, la pyrrolidone-2, le lactate d'éthyle, les glycérides en C₈-C₁₀ polyoxyéthylénés, le poly-éthylène glycol 20 glycéryle laurate et le diméthylacétamide.

## Patentansprüche (Patentansprüche für folgende(n) Vertragsstaat(en): AT, BE, CH, DE, DK, FR, GB, IT, LI, NL, PT, SE)

1. Dermatologische Zusammensetzung für die Behandlung von Onychomykosen, die in Form einer Lösung vorliegt und enthält
- eine wirksame Menge Terbinafinhydrochlorid als Antifungi-Mittel,
- eine wirksame Menge mindestens eines Agens, das die Penetration der keratinisierten Ungual-Platte des Nagels bzw. der Kralle bzw. des Hufs durch das genannte Antifungi-Mittel fördert, und
- ein Lösungsmittel-Medium für das genannte Antifungi-Mittel,
dadurch gekennzeichnet, daß das genannte Lösungsmittel-Medium Wasser im Gemisch mit mindestens einem Dritt-Lösungsmittel, ausgewählt aus C₂-C₈-Alkanolen mit gerader oder verzweigter Kette, enthält und daß das (die) genannte(n) Penetrationsmittel ausgewählt wird aus der Gruppe der Glycole, der Glycolmonoether, der Glycoldiether, Dimethylsulfoxid, Caprolactam, Dimethylisosorbit, Isopropylidenglycerin, Dimethylimidazolidinon, N-Methyl-2-pyrrolidon, 2-Pyrrolidon, Ethyllactat, der C₈-C₁₀-Polyoxyethylen-glyceride, Polyethylenglycol-20-glyceryllaurat und Dimethylacetamid.

2. Zusammensetzung nach Anspruch 1, dadurch gekennzeichnet, daß die Konzentration an Antifungi-Mittel 2 bis 30 Gew.-%, bezogen auf das Gesamtgewicht der genannten Zusammensetzung, beträgt.

3. Zusammensetzung nach Anspruch 1 oder 2, dadurch gekennzeichnet, daß die Glycole ausgewählt werden aus der Gruppe Propylenglycol, Butylenglycol, Hexylenglycol, Ethylenglycol und der Polyethylenglycole; der Glycolmonoether, ausgewählt aus der Gruppe der Ethylenglycolmonoether und der Propylenglycolmonoether; und der Glycoldiether, ausgewählt aus der Gruppe Propylenglycoldimethylether und Dipropylenglycoldimethylether.

4. Zusammensetzung nach einem der Ansprüche 1 bis 3, dadurch gekennzeichnet, daß es sich bei dem Penetrationsmittel um Dipropylenglycoldimethylether handelt.

5. Zusammensetzung nach einem der Ansprüche 1 bis 4, dadurch gekennzeichnet, daß die Konzentration an Penetrationsmittel(n) 1 bis 60 Gew.-%, bezogen auf das Gesamtgewicht der Zusammensetzung, beträgt.

6. Zusammensetzung nach einem der Ansprüche 1 bis 5, dadurch gekennzeichnet, daß das Wasser und das (die) Dritt-Lösungsmittel(n) jeweils in einer Menge von 5 bis 30 Gew.-% bzw. von 10 bis 90 Gew.-%, bezogen auf das Gesamtgewicht der Zusammensetzung, vorliegen.

7. Zusammensetzung nach einem der Ansprüche 1 bis 6, dadurch gekennzeichnet, daß sie mindestens ein Additiv enthält, ausgewählt aus der Gruppe der Konservierungsmittel, der Antioxidationsmittel, der Färbemittel, der Füllstoffe, der Pigmente und der Polymeren, die das Abfließen der Zusammensetzung vor der Penetration verhindern sollen.

8. Zusammensetzung nach einem der Ansprüche 1 bis 7, dadurch gekennzeichnet, daß sie in Form einer Lotion oder eines fließfähigen Gels vorliegt.

## Patentansprüche (Patentansprüche für folgende(n) Vertragsstaat(en): ES, GR)

1. Verfahren zur Herstellung einer dermatologischen Zusammensetzung für die Behandlung von Onychomykosen, dadurch gekennzeichnet, daß man eine wirksame Menge Terbinafinhydrochlorid als Antifungi-Mittel in einem Gemisch von Wasser und einem Dritt-Lösungsmittel, ausgewählt aus den C₂-C₈-Alkanolen mit gerader oder verzweigter Kette, unter Rühren löst und daß man allmählich ein oder mehrere Penetrationsmittel, ausgewählt aus der Gruppe der Glycole, der Glycolmonoether, der Glycoldiether, Dimethylsulfoxid, Caprolactam, Dimethylisosorbit, Isopropylidenglycerin, Dimethylimidazolidinon, N-Methyl-2-pyrrolidon, 2-Pyrrolidon, Ethyllactat, der C₈-C₁₀-Polyoxyethylenglyceride, Polyethylenglycol-20-glyceryllaurat und Dimethylacetamid in einer wirksamen Menge zugibt, wobei man eine Lösung erhält.

2. Verfahren nach Anspruch 1, dadurch gekennzeichnet, daß die Menge des (der) Antifungi-Mittel(s) so ist, daß seine (ihre) Konzentration in der Lösung 2 bis 30 Gew.-%, bezogen auf das Gesamtgewicht der Lösung, beträgt.

3. Verfahren nach Anspruch 1 oder 2, dadurch gekennzeichnet, daß die Glycole ausgewählt werden aus der Gruppe Propylenglycol, Butylenglycol, Hexylenglycol, Ethylenglycol und der Polyethylenglycole; der Glycolmonoether, ausgewählt aus der Gruppe der Ethylenglycolmonoether und der Propylenglycolmonoether; und der Glycoldiether, ausgewählt aus der Gruppe Propylenglycoldimethylether und Dipropylenglycoldimethylether.

4. Verfahren nach einem der Ansprüche 1 bis 3, dadurch gekennzeichnet, daß es sich bei dem Penetrationsmittel um Dipropylenglycoldimethylether handelt.

5. Verfahren nach einem der Ansprüche 1 bis 4, dadurch gekennzeichnet, daß die erhaltene Lösung 1 bis 60 Gew.-% Penetrationsmittel, bezogen auf das Gesamtgewicht der Zusammensetzung, enthält.

6. Verfahren nach einem der Ansprüche 1 bis 5, dadurch gekennzeichnet, daß die erhaltene Lösung 5 bis 30 Gew.-% Wasser und 10 bis 90 Gew.-% Alkanol, bezogen auf das Gesamtgewicht der Zusammensetzung, erhält.

7. Verfahren nach einem der Ansprüche 1 bis 6, dadurch gekennzeichnet, daß man in einer beliebigen Stufe des Verfahrens mindestens ein Additiv zugibt, das ausgewählt wird aus der Gruppe der Konservierungsmittel, der Antioxidationsmittel, der Färbemittel, der Füllstoffe, der Pigmente und der Polymeren, die das Abfließen der Zusammensetzung vor der Penetration verhindern sollen.

8. Dermatologische Zusammensetzung für die Behandlung von Onychomykosen, die in Form einer Lösung vorliegt und enthält
- eine wirksame Menge Terbinafinhydrochlorid als Antifungi-Mittel,
- eine wirksame Menge mindestens eines Agens, das die Penetration der keratinisierten Ungual-Platte des Nagels bzw. der Kralle bzw. des Hufs durch das genannte Antifungi-Mittel fördert, und
- ein Lösungsmittel-Medium für das genannte Antifungi-Mittel,
dadurch gekennzeichnet, daß das genannte Lösungsmittel-Medium Wasser im Gemisch mit mindestens einem Dritt-Lösungsmittel, ausgewählt aus C₂-C₈-Alkanolen mit gerader oder verzweigter Kette, enthält und daß das (die) genannte(n) Penetrationsmittel ausgewählt wird aus der Gruppe der Glycole, der Glycolmonoether, der Glycoldiether, Dimethylsulfoxid, Caprolactam, Dimethylisosorbit, Isopropylidenglycerin, Dimethylimidazolidinon, N-Methyl-2-pyrrolidon, 2-Pyrrolidon, Ethyllactat, der C₈-C₁₀-Polyoxyethylen-glyceride, Polyethylenglycol-20-glyceryllaurat und Dimethylacetamid.
